# EUROPEAN PATENT APPLICATION

(11) **EP 2 572 657 A1**
(43) Date of publication of application: **27.03.2013**
(21) Application number: 12185455.8
(22) Date of filing: 21.09.2012
(51) Int. Cl.: A61B 17/17, A61B 1/00

(54) **Arthroscope guide**

(30) Priority: 21.09.2011 EP 11182182
(71) Applicant: ARTHREX MEDIZINISCHE INSTRUMENTE GmbH, D-85757 Karlsfeld (DE)
(72) Inventor: Krupp, Stefan, 80469 Munich (DE); Bodinger, Benedikt, 85232 Günding (DE)
(74) Representative: Lohr, Georg

(57) **Abstract**

A guide for guiding the distal end of an arthroscope above the surface of a bone plate is disclosed. The guide is located on top of the bone plate. It has a body with cutouts and/or grooves to guide the arthroscope. Furthermore the arthroscope has a ball shaped slider close to its distal end. This slider is adapted to interface with the guide and allows movement of the distal end of the arthroscope along the cutouts or grooves.

## Description

### Field of the invention

The present invention relates to arthroscopic instruments and implants, specifically to arthroscopes and implant plates.

### Background of the invention

For fixing fractures of bones or fixing and stabilizing bones after an osteotomy bone plates are used. The classic way to apply such a plate is completely opening the operating field. This allows free access to the bone and the plate, so the plate and the screws for fixing the plate can be located precisely. The disadvantages are risk of infection, injury of nerves and vessels and large scars. Therefore it is preferred to insert the plate through a small incision and push it between the bone and the surrounding tissue. The screws are placed through individual small incisions through the tissue. The drawback of this technique is the hidden plate. As the plate is covered by the tissue, precise location of the screws is very difficult.

An bone plates for stabilizing a fracture is disclosed in US patent application publication 2005/0021033 A1. It has a surface adapted to the bone and holes for inserting screws to fix the plate into the bone. A guide for locating a screwdriver at an implant plate is disclosed in CN 201 283 001 Y. Bone plates and a system for adjusting the same are located in EP 1 808 137 A1. Instruments and methods for holding a bone plate are disclosed in US 2005/0283155.

### Summary of the invention

The problem to be solved by the invention is to provide means for improving positioning of screws within a bone plate under the surrounding tissue.

Solutions of the problem are described in the independent claims. The dependent claims relate to further improvements of the invention.

To improve placement of the screws an arthroscope may be inserted through the incision for locating the screw tips. Such an arthroscope may be inserted between the bone plate and the surrounding tissue.

A first embodiment comprises a guide which can be placed on the bone plate. This guide has a surface structure which supports sliding of the distal end of an arthroscope into predetermined directions, preferably into a first direction, which preferably is approximately parallel to the main axis of the plate. The surface structure preferably is a groove, a cutout or a slot. This surface structure is preferably oriented roughly in parallel to the main axis of the plate, or an axis along the center of a bone to which the plate is attached. This prevents the arthroscope slipping away from the bone plate and the surgeon from losing view of the holes and the screws. Preferably the guide is made of plastic or resin. Without such a guide it is extremely difficult to precisely position the distal end of the arthroscope for viewing a specific section of the surface of the plate with a hole and the screw penetrating the tissue and entering the hole. The guide may be fixed to the plate to prevent movement of the guide versus the plate preferably in a direction perpendicular to the first direction.

It is furthermore preferred, if the guide can be clipped to the plate or is at least held to the plate by friction. The guide has preferably side surfaces to allow alignment with the plate. Alignment may also be done by engagement with at least one of the holes of the plate or other structures of the plate. The guide may sit loose on the plate and may only be held by gravity or the pressure of the surrounding tissue against the bone. The guide may have further means for simplifying insertion of the guide with or without the plate into the space between the bone and the surrounding tissue. Such means may be at least one taper. It is preferred, if the guide can be attached to the plate outside of the body, before insertion of the plate into the body.

The optical systems of arthroscopes have only a limited range of focus. Therefore there must be some minimum distance from the lens of an arthroscope which is usually at the distal end of the instrument to an object to be viewed. Accordingly it is preferred that the guide holds the arthroscope in such a distance from the bone plate that a significant part of the field of view is within the focus range and parts of the bone plate like its surface or holes can be clearly seen.

Another embodiment comprises a method for improving placement of the screws by inserting an arthroscope through the incision for locating the screw tips or a any other medical implant and/or instrument. Such an arthroscope may be inserted between the bone plate and the surrounding tissue.

A further embodiment comprises an arthroscope having a slider at its distal end. This slider may comprise of an enlarged diameter section. It may have a cylindrical or preferably a ball shape. It may also have any spherical or elliptical shape. The diameter of the slider is preferably in a range between 5 mm to 15 mm. It is further preferred if the enlarged diameter section is about 5 mm to 30 mm distant from the distal end of the arthroscope. The slider allows sliding of the distal end of the arthroscope over the plate along the surface structure while maintaining sufficient distance to the surface of the plate for viewing the surface of the plate within the focus range and without getting stuck into the holes of the plate during movement. Herein the term arthroscope relates to an endoscope suitable for viewing the space between the plate and the surrounding tissue.

In another embodiment the arthroscope may be contained within a sheath. The sheath may have a slider.

In a preferred embodiment an arthroscopic instrument set comprises of a guide and an arthroscope being adapted to each other. Here the slider is adapted to interface with the guide e.g. to slide within the groove of the guide.

### Brief Description of the Drawings

In the following the invention will be described by way of example, without limitation of the general inventive concept, on examples of embodiment with reference to the drawings.
Figure 1 shows an embodiment of a slider and a guide
Figure 2 shows a side view of a slider and a guide.
Figure 3 shows a guide attached to a plate.
Figure 4 shows a side view of a guide attached to a plate.
Figure 5 a first cross-section view.
Figure 6 shows a second cross-section view.
Figure 7 shows a further embodiment of a guide.

While the invention is susceptible to various modifications and alternative forms, specific embodiments thereof are shown by way of example in the drawings and will herein be described in detail. It should be understood, however, that the drawings and detailed description thereto are not intended to limit the invention to the particular form disclosed, but on the contrary, the intention is to cover all modifications, equivalents and alternatives falling within the spirit and scope of the present invention as defined by the appended claims.

### Detailed description of the preferred embodiments

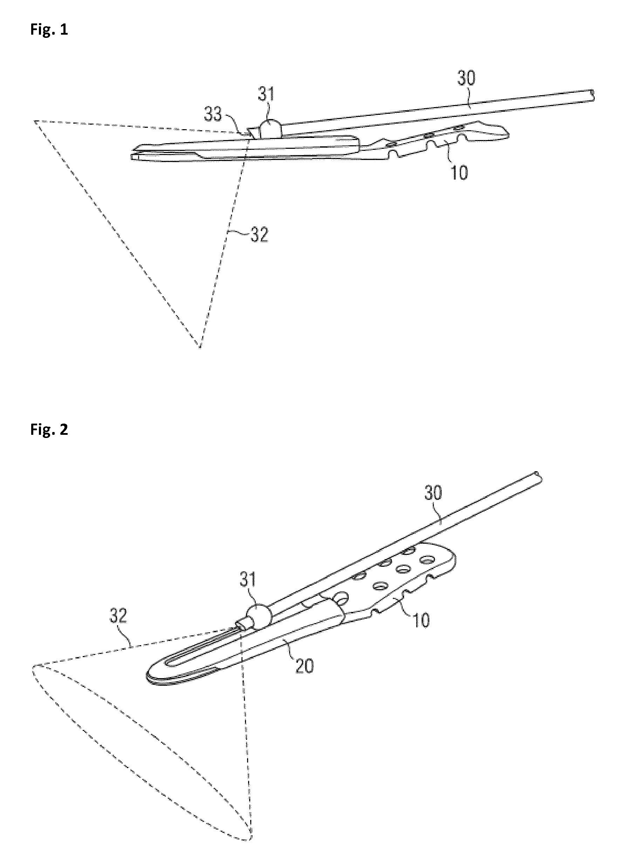

Figure 1 shows an embodiment of a slider and a guide. Attached to a bone plate 10 is a guide 20. Both, the bone plate and the guide are inserted into the space between the bone, not shown here and surrounding tissue like muscle, also not shown here. According to the orientation of this figure the bone would be below the plate, while the tissue would be above the plate and the guide. Furthermore an arthroscope 30 is inserted into the space between the bone and surrounding tissue, above the plate. Due to the tissue surrounding the bone, the arthroscope may only be inserted roughly parallel to the plate. This arthroscope allows an orthopedic surgeon to locate individual holes in the bone plate and to position screws precisely to hold the bone plate to the bone. The arthroscope is guided by the guide above the surface of the bone plate. This prevents the arthroscope slipping away from the bone plate and the surgeon from losing view of the holes and the screws. The guide preferably holds the arthroscope in the distance from the plate to keep a significant part of the objects in the field of view 32 within the focus range.

This figure shows a preferred embodiment of an arthroscope 30, having a slider 31 close to its distal end. The slider is guided by the guide and allows precise location in a predetermined distance above the bone plate. After at least one, preferably all screws have been inserted into the bone plate, the guide 20 may easily be removed by pulling it out from the space between the bone and surrounding tissue. It is obvious that according to an embodiment the arthroscope may not necessarily have a slider. In this case it is preferred, if the guide is adapted to the smaller diameter of the arthroscope compared to the diameter of the slider. Furthermore, according to another embodiment the arthroscope may have a slider holding a predetermined distance to the bone plate, even without using a guide and allowing sliding without sticking to holes or screw heads.

Figure 2 shows a side view of a bone plate 10 with a guide 20 and an arthroscope 30. Here also the lens 33 being located at the distal end of the arthroscope is indicated. It is obvious that even a slider 31 without the guide 20 allows better sliding of the arthroscope over the surface of the bone plate. While the distal end of the arthroscope with its lens usually has a sensitive and comparatively sharp edge which may easily stick to a hole or a screw head on the surface of the bone plate when sliding over the surface of the bone plate the larger radius of slider 31 allows easy sliding over the surface of the bone plate.

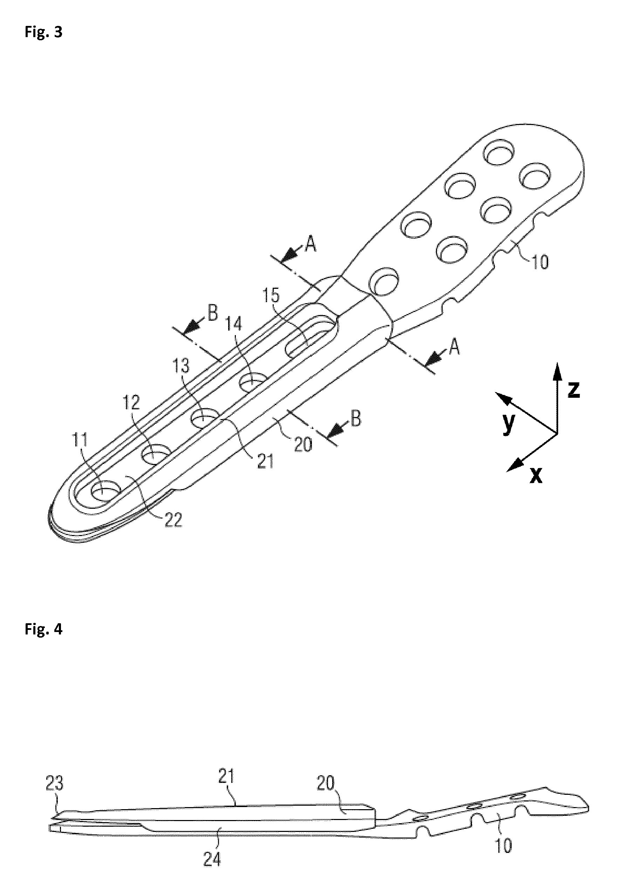

Figure 3 shows a guide attached to a plate. Here holes 11, 12, 13, 14 and 15 in the plate are shown. They are within a cutout 22 of the top surface 21 of the guide 20. The holes of the bone plate can easily be watched, as they are close to the incision, through which the bone plate was inserted. Of course the guide may also cover larger areas of the surface of bone plate 20. The guide may even cover the whole length or the whole surface of the plate. The guide may also have two or more cutouts or grooves for guiding the arthroscope over multiple rows of screws, preferably multiple parallel rows of screws. The guide guides the arthroscope for a linear movement along the x-axis. For this purpose the guide is fixed to the plate to prevent the movement of the guide versus the plate in the direction of the y-axis.

Figure 4 shows a side view of a guide attached to a plate. Sidewall 24 helps holding and positioning the guide on the bone plate. To simplify insertion and/or removal of the bone plate together with the guide into the space between the bone and surrounding tissue, it is preferred, if the guide has at least one taper 23 at at least one of its ends.

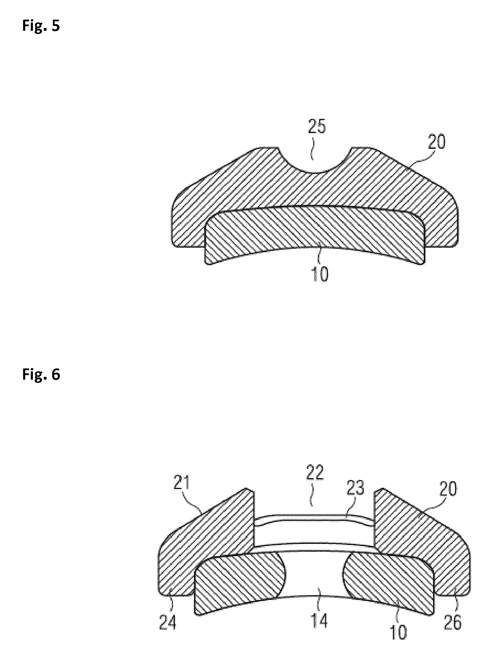

Figure 5 shows a first cross-section view at location A-A of figure 4. Here a groove 25 is shown for guiding the arthroscope. It is preferred, if the diameter and/or the depth of the groove is adapted to be diameter of the arthroscope.

Figure 6 shows a second cross-section view at location B-B of figure 4. It further shows the cutout 22 allowing free access to screw hole 14. Furthermore taper 23 located at the end of the bone plate is shown.

It will be appreciated to those skilled in the art having the benefit of this disclosure that this invention is believed to provide a guide and the slider for precise location of the arthroscope above the bone plate. Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as the presently preferred embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

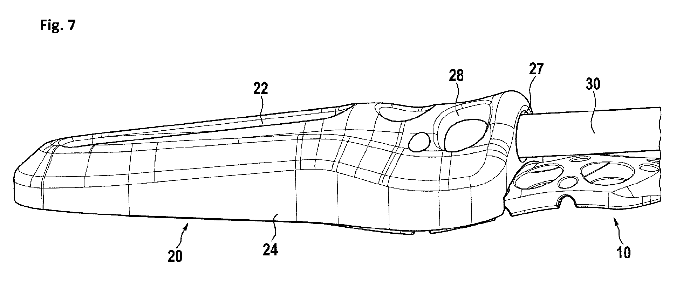

Figure 7 shows a further embodiment of a guide. This guide is at least covering partially the bone plate 10. This results in a better guidance of the arthroscope 30 in preferably at least two axis. The arthroscope is inserted through arthroscope hole 27 and guided essentially parallel to the bone plate 10 along the x-axis. The arthroscope may not only be guided by at least one of the sidewalls 24 to prevent deviation of the arthroscope in the direction of the y-axis. It may also be guided by the top side having a cutout 22 to prevent deviation of the arthroscope in the direction of the z-axis. In this case, the width of the cutout 22 may be smaller than the diameter of the arthroscope to prevent slipping out of the arthroscope. Most preferably the sidewalls of the guide have a distance at the inner side of the guide which is equal or slightly larger than the diameter of the arthroscope. There may be further holes for inserting screws into the bone plate.

### List of reference numerals

- 10: bone plate
- 11 ... 15: holes in plate
- 20: guide
- 21: body
- 22: cutout
- 23: taper
- 24, 26: sidewall
- 25: groove
- 27: arthroscope hole
- 28: hole
- 30: arthroscope
- 31: slider
- 32: field of view
- 33: lens

## Claims

1. Guide (20) for guiding the distal end of an arthroscope (30) above the surface of a bone plate (10) into a first direction (x) comprising of a body (21) to be located on the bone plate, the body having at least
- one cutout (22) and/or at least one groove (25) adapted to guide the distal end of an arthroscope, and
- at least one means (24) to fix the body to the bone plate (10) such to prevent a movement of the body perpendicular to the first direction.

2. Guide according to claim 1,
**characterized in that**
the body (21) has at least two sidewalls (24, 26) for locating and holding the guide on the bone plate.

3. Guide according to claim 1 or 2,
**characterized in that**
at least one cutout (22) and/or at least one groove (25) is adapted to the diameter of the distal end of the arthroscope.

4. Guide according to any one of claims 1 to 3,
**characterized in that**
the body (21) is adapted to guide the arthroscope (30) in a predetermined distance to the surface of the bone plate (10).

5. Guide according to any one of claims 1 to 4,
**characterized in that**
the guide comprises of resin or plastic material.

6. Guides according to any one of claims 1 to 5,
**characterized in that**
the body (21) closely fits to at least part of the surface of the bone plate (10).

7. Arthroscopic instrument set comprising of
- a guide (20) according to any one of the previous claims,
- an arthroscope (30) having at least one slider (31) located close to the distal end of the arthroscope for sliding within the at least one cutout (22) and/or at least one groove (25) of the guide.

8. Arthroscopic instrument set according to claim 7,
**characterized in that**
the body (21) has at least two sidewalls (24, 26) for locating and holding the guide on the bone plate.

9. Arthroscopic instrument set according to claim 7 or 8,
**characterized in that**
at least one cutout (22) and/or at least one groove (25) is adapted to the diameter of the distal end of the arthroscope.

10. Arthroscopic instrument set according to any one of claims 7 to 9,
**characterized in that**
the body (21) is adapted to guide the arthroscope (30) in a predetermined distance to the surface of the bone plate (10).

11. Arthroscopic instrument set according to any one of claims 7 to 10,
**characterized in that**
the guide comprises of resin or plastic material.

12. Arthroscopic instrument set according to any one of claims 7 to 11,
**characterized in that**
the body (21) closely fits to at least part of the surface of the bone plate (10).

13. Arthroscope (30) having at least one slider (31) located close to the distal end of the arthroscope for sliding on the surface of a bone plate or for sliding on a guide according to any one of claims 1 to 6.

14. Arthroscope (30) according to claim 13,
**characterized in that**
the slider (31) is ball shaped.
